# FASCICULE DE BREVET EUROPEEN

(11) **EP 2 170 466 B1**
(45) Date de publication et mention de la délivrance du brevet: **22.10.2014**
(21) Numéro de dépôt: 08827559.9
(22) Date de dépôt: 01.08.2008
(51) Int. Cl.: A61K 8/49, A61Q 19/06, A61K 8/60

(54) **UTILISATION D'HESPÉRIDINE OU DE L'UN DE SES DÉRIVÉS POUR LA PRÉVENTION ET/OU LE TRAITEMENT DES PEAUX RELÂCHÉES**
VERWENDUNG VON HESPERIDIN ODER EINEM DERIVAT DAVON ZUR PRÄVENTION UND/ODER BEHANDLUNG VON HAUTERSCHLAFFUNGEN
USE OF HESPERIDIN OR OF A DERIVATIVE THEREOF FOR THE PREVENTION AND/OR TREATMENT OF SLACKENED SKIN

(30) Priorité: 02.08.2007 FR 0756898
(43) Date de publication de la demande: 07.04.2010
(73) Titulaire: L'Oréal, 75008 Paris (FR); Nestec S.A., 1800 Vevey (CH)
(72) Inventeur: GUENICHE, Audrey, F-92500 Rueil Malmaison (FR); CASTIEL, Isabelle, F-06200 Nice (FR)
(74) Mandataire: Le Coupanec, Pascale A.M.P.
(86) Numéro de dépôt international: PCT/FR2008/051453
(87) Numéro de publication internationale: WO 2009/022077

(56) Documents cités:
- EP-A- 1 327 438
- WO-A-01/64177
- WO-A-03/068141
- WO-A-2005/000831
- FR-A- 2 578 165
- FR-A- 2 778 663
- FR-A- 2 869 229

## Description

La présente invention se rapporte à la prévention et/ou au traitement des peaux qualifiées de peaux relâchées.

La peau humaine est constituée par trois compartiments, à savoir un compartiment superficiel, l'épiderme, le derme et un compartiment profond, l'hypoderme.

Le derme fournit à l'épiderme un support solide. C'est également son élément nourricier. Il est principalement constitué de fibroblastes et d'une matrice extracellulaire composée elle-même principalement de collagène, d'élastine et d'une substance dite substance fondamentale, composants synthétisés par les fibroblastes.

La matrice extracellulaire (MEC) de la peau humaine est constituée de diverses macromolécules responsables de la résistance mécanique de la peau, de sa souplesse, de sa tonicité et de son élasticité, ainsi que des fonctions physiologiquement importantes (hydratation, thermorégulation et régulation de la perméabilité de la peau).

Les macromolécules de la MEC ont été arbitrairement classées en quatre familles. Les deux premières sont constituées de macromolécules fibreuses et structurales, les collagènes et l'élastine, alors que les deux autres sont des glycoconjugués (glycoprotéines et protéoglycanes).

Les collagènes représentent 70 % des protéines de la MEC. De nombreux types de collagène constituent la MEC, dont notamment les collagènes interstitiels (de type I, II, III) de structure fibrillaire, produits essentiellement par les fibroblastes, et responsables de la cohésion, de la rigidité et de la résistance mécanique, les collagène des lames basales (de type IV) synthétisés par les cellules adjacentes et dans la peau par les kératinocytes et jouant notamment un rôle mécanique, les collagènes formant des fibrilles d'ancrage de la membrane basale (lien derme-épiderme) exprimées par les kératinocytes épidermiques (de type VII) ou encore les collagènes de type V et XII, non fibrillaires, qui ne sont pas retrouvées au niveau cutané.

En particulier, le collagène de type IV est spécifique de la membrane basale et les collagènes de type VI et VII (collagènes microfibrillaires) sont retrouvés au niveau de la jonction dermoépidermique.

La fermeté et la résistance à la pression de la peau, et notamment sa tonicité, dépendent surtout des fibres de collagène de la membrane basale et de la jonction dermoépidermique.

Plus précisément, les fibres de collagène sont constituées de fibrilles scellées les unes aux autres, formant ainsi plus de dix types de structures différentes. La solidité du derme est en grande partie due à l'enchevêtrement des fibres de collagène tassées les unes contre les autres en tout sens. Les fibres de collagène participent ainsi à l'élasticité et à la tonicité de la peau et/ou des muqueuses.

Au cours du temps, lors de modifications de poids (tels que dans les régimes amincissants, les grossesses...) ou de modifications hormonales (tels que lors de la ménopause), les fibres de collagène perdent progressivement leur densité, leur tension et leur alignement régulier.

Naturellement, les fibres de collagène sont constamment renouvelées mais ce renouvellement diminue avec l'âge ce qui entraîne un amincissement du derme. Cet amincissement du derme peut être également dû à des causes pathologiques, comme par exemple l'hypersécrétion d'hormones corticoïdes, certaines pathologies ou encore des carences vitaminiques.

De même, il est également admis que des facteurs extrinsèques comme les rayons ultraviolets, le tabac ou certains traitements (glucocorticoïdes, vitamine D et dérivés par exemple) ont également un effet sur la peau et son taux de collagène.

Par ailleurs, les fibres de collagène sont sensibles à certaines enzymes appelées collagénases.

Les collagénases font partie d'une famille d'enzymes appelées métalloprotéinases qui sont elles-mêmes les membres d'une famille d'enzymes protéolytiques (endoprotéases ou endopeptidases). Leur surexpression chez l'homme et leur activation est liée à de nombreux processus, parfois pathologiques, qui impliquent la destruction et le remodelage de la matrice.

L'exposition prolongée aux rayonnements ultraviolets, particulièrement aux rayonnements de type A et B, a ainsi pour effet une stimulation de l'expression des collagénases, particulièrement de la MMP1, constituant une des composantes du vieillissement cutanée photo-induit.

Par ailleurs, à la ménopause, les principales modifications concernant le derme sont une diminution du taux de collagène et de l'épaisseur dermique. Cela entraîne chez la femme ménopausée un relâchement de la peau et/ou des muqueuses du fait que la peau présente une élasticité diminuée.

Enfin, chez les sujets en surpoids, et plus particulièrement lors d'une prise de poids, les adipocytes ont tendance à augmenter rapidement de volume (du fait d'un stockage de quantités croissantes de lipides). La forte pression exercée par les adipocytes sur le derme provoque rapidement une déformation de la surface de la peau.

A terme, les fibres dégénèrent et la peau perd ses structures fondamentales, ce qui se traduit par une altération de ses propriétés viscoélastiques ou biomécaniques (perte de fermeté, de tonicité, d'élasticité).

Sur le plan biologique, chez un sujet ne subissant pas de variation de poids conséquente, lorsque les fibroblastes sont soumis à une tension tissulaire normale, ils synthétisent activement du collagène, de l'élastine, des glycosaminoglycanes, molécules fondamentales qui contribuent à renforcer les tissus de soutien de la peau. De façon analogue, les adipocytes surchargés de lipides exercent aussi une tension sur le derme, entraînant une surproduction de collagène jusqu'à la fibrose. Cela se traduit, au plan clinique, par une peau plus consistance et tendue.

En revanche, lors d'une perte de poids, et notamment au cours des régimes amincissants, le déstockage rapide des adipocytes entraîne une diminution importante de la tension exercée par l'hypoderme sur les tissus de soutien. Par conséquent, le derme n'étant plus sous tension, le tissu conjonctif perd progressivement de sa cohésion : perte d'attache des fibroblastes au collagène, diminution de la quantité de néocollagène, distension des fibres d'élastine, dépolymérisation des protéoglycanes.

Dès lors, les fibroblastes sont en moins grande interaction avec les fibres de la matrice extracellulaire.

En conséquence, il ressort de ce qui précède que de nombreux facteurs entraînent ainsi la dégradation des fibres de collagène, avec toutes les conséquences que l'on peut envisager sur la structure, la tonicité et/ou la fermeté de la peau et/ou des muqueuses.

On comprend alors l'importance du collagène et des glycosaminoglycanes dans la structure des tissus, particulièrement de la peau et/ou des muqueuses, et l'importance qu'il y a à combattre sa dégradation pour ainsi lutter contre le vieillissement, qu'il soit chronologique ou photoinduit, et ses conséquences, notamment sur l'amincissement du derme et/ou la dégradation des fibres de collagène, cette dernière conséquence entraînant en particulier l'apparence de peau molle, contre laquelle l'objet de la présente invention est précisément de lutter.

L'utilisation de flavonoïdes, dont l'hespéridine, pour augmenter la prolifération cellulaire, et traiter notamment les cicatrices, est déjà connue de WO 03/057210.

WO 2005/058255 décrit par ailleurs l'action de flavanones particuliers, dont l'hespéridine, pour traiter les désordres de la peau et des cheveux, en particulier *via* les propriétés cytoprotectives et anti-inflammatoires de ceux-ci.

Le document EP 0 774 249 tire profit d'un effet synergique constaté d'une combinaison de flavanones spécifiques, d'une part, ou d'une combinaison de flavanones spécifiques avec un céramide particulier, d'autre part, sur la différenciation des kératinocytes.

Le document WO 01/64177 décrit l'utilisation (par voie topique) d'isoflavones naturelles extraites du soja, c'est-à-dire notamment de la génistine, la daidzine, la daidzéine, la génistéine, la glycitéine et de leurs formes acétyles et malonyles pour le traitement cosmétique de la cellulite ou pour la fermeté de la peau.

WO 03/068141 A vise l'utilisation par voie topique d'une association synergique d'au moins deux actifs, pouvant notamment comprendre l'hespéridine, pour prévenir les symptômes du relâchement cutané, et plus particulièrement diminuer les poches et les cernes sous les yeux.

Le document FR-A-2 869 229 enseigne, de façon générale l'utilisation par voie topique d'un inducteur des enzymes UGT pour protéger et/ou améliorer l'état de la peau.

Le document EP-A-1 327 438 décrit l'utilisation cosmétique d'acide ursolique, d'acide oléanique et/ou de flavonoïde, et en particulier de rutine et de naringine, pour le traitement du vieillissement cutané.

Le document FR-A-2 578 165 vise l'utilisation de pommades à action anti-cellulitique comprenant un ou plusieurs bio-flavonoïdes associés à la teinture mère des Hedera hélix et à la teinture mère de Fucus vesiculorum.

Aucun des quatre derniers documents cités ne divulgue l'utilisation d'hespéridine ou de l'un de ses dérivés par voie orale.

Le document FR-A-2 778 663 concerne l'utilisation d'esters de flavonoïdes dans des compositions cosmétiques ou pharmaceutiques, néanmoins ce document ne mentionne pas les dérivés d'hespéridine considérés selon la présente invention.

Pour leur part, les inventeurs ont constaté que l'hespéridine et certains de ses dérivés s'avèrent posséder un effet bénéfique au niveau de la tonicité de la peau. Son administration raffermit la peau, et prévient efficacement son relâchement.

Les dérivés d'hespéridine particuliers considérés selon l'invention sont choisis parmi ses formes aglycones, ses formes chalcones, ses formes glycosylées et ses formes mithylies, ainsi que ses formes sulfatées ou glucuronidées qui sont retrouvées comme produits du métabolisme dans la circulation sanguine.

La présente invention concerne ainsi l'utilisation cosmétique par voie orale d'une quantité efficace d'hespéridine ou de l'un de ses dérivés particuliers considérés selon l'invention comme agent raffermissant de la peau.

La présente invention concerne également l'utilisation cosmétique par voie orale d'une quantité efficace d'hespéridine ou de l'un de ses dérivés particuliers considérés selon l'invention comme agent pour prévenir et/ou traiter les peaux relâchées.

Contre toute attente, les inventeurs ont en effet constaté que l'hespéridine et ses dérivés considérés selon l'invention, manifestent avantageusement une activité bénéfique de restructuration de la peau, améliorant ainsi sa tonicité, sa fermeté et son lissage.

Ainsi, l'utilisation par voie orale d'une quantité efficace d'hespéridine ou de l'un de ses dérivés particuliers considérés selon l'invention permet en particulier de traiter les vergetures ou de prévenir leur apparition.

Les vergetures se présentent comme des cicatrices en forme de stries légèrement creusées de coloration rosée ou nacrée. Elles se localisent principalement aux endroits où la peau est étirée : ventre, hanches, cuisses, fesses, seins.

L'invention a également pour objet l'utilisation cosmétique par voie orale d'une quantité efficace d'hespéridine ou de l'un de ses dérivés particuliers considérés selon l'invention, comme agent pour maintenir et/ou restaurer la tonicité et/ou la fermeté de la peau.

Elle concerne aussi l'utilisation cosmétique particuliers considérés selon l'invention par voie orale d'une quantité efficace d'hespéridine ou de l'un de ses dérivés particuliers considérés selon l'invention, comme agent pour atténuer l'aspect de la cellulite et de la peau d'orange.

Elle concerne également l'utilisation cosmétique par voie orale d'une quantité efficace d'hespéridine ou de l'un de ses dérivés particuliers considérés selon l'invention comme agent pour traiter les vergetures ou prévenir leur apparition.

Elle concerne également l'utilisation cosmétique par voie orale d'une quantité efficace d'hespéridine ou de l'un de ses dérivés particuliers considérés selon l'invention, à titre d'agent restructurant et/ou d'agent anti-relâchement de la peau et/ou des muqueuses.

L'invention a encore pour objet l'utilisation d'une quantité efficace d'hespéridine ou de l'un de ses dérivés particuliers considérés selon l'invention pour la préparation d'une composition, notamment cosmétique et/ou dermatologique, pour voie orale, destinée à prévenir et/ou traiter les peaux relâchées.

L'invention a également pour objet l'utilisation d'une quantité efficace d'hespéridine ou de l'un de ses dérivés particuliers considérés selon l'invention pour la préparation d'une composition, notamment cosmétique et/ou dermatologique, destinée à prévenir et/ou traiter les désordres de la peau liés à l'atrophie cutanée et/ou à une synthèse de collagène détériorée et/ou à une surexpression de métalloprotéases matricielles.

Selon un autre de ses aspects, la présente invention concerne l'utilisation cosmétique par voie orale d'une quantité efficace d'hespéridine ou de l'un de ses dérivés particuliers considérés selon l'invention, pour prévenir et/ou traiter une dégénérescence des tissus conjonctifs liée à une anomalie de la production de collagène, en particulier de collagène de type IV et VII.

L'invention concerne également un procédé cosmétique de traitement et/ou de prévention des peaux relâchées et/ou des vergetures comprenant l'administration, par voie orale, à un sujet d'une quantité efficace d'hespéridine ou de l'un de ses dérivés particuliers considérés selon l'invention.

Selon un mode de réalisation, ledit sujet peut être une femme enceinte.

Selon un autre mode de réalisation, ledit sujet peut être un sujet suivant un régime amincissant.

Au sens de la présente invention, le terme « prévenir » entend le fait de diminuer le risque de survenue d'un phénomène.

Par « quantité efficace », on entend au sens de la présente invention une quantité suffisante pour obtenir l'effet attendu.

L'hespéridine ou l'un de ses dérivés particuliers considérés selon l'invention peuvent être formulés dans des compositions cosmétiques ou dermatologiques.

Selon un mode de réalisation, l'utilisation ou le procédé selon l'invention peut comprendre l'administration d'une quantité efficace d'hespéridine ou de l'un de ses dérivés par voie, orale.

La voie orale présente l'avantage d'agir de façon globale sur l'ensemble de la peau et ce dans ses couches profondes (derme, hypoderme), suivant un mode d'administration rapide et peu contraignant. En effet, les métabolites et autres nutriments actifs sont en particulier distribués au sein de la matrice dermique par le biais de la circulation sanguine. La voie orale ou l'administration par patch présentent également l'avantage d'un mode d'administration rapide et peu contraignant.

Selon un mode de réalisation préféré, l'utilisation cosmétique selon l'invention est donc effectuée par voie orale et le procédé selon l'invention comprend l'administration par voie orale de ladite quantité efficace d'hespéridine ou de l'un de ses dérivés particuliers considérés selon l'invention.

### Hespéridine

L'hespéridine appartient à la famille des flavanones, qui sont des composés glucosides naturels retrouvés principalement dans les agrumes, c'est-à-dire les fruits du genre *Citrus,* tels que par exemple les oranges, les citrons, les oranges amères ou encore les raisins.

Elles sont présentes majoritairement dans la peau des agrumes mais sont également retrouvées en grandes quantités dans la pulpe, et donc dans le jus des agrumes.

L'hespéridine est un composé glucosylé comprenant un noyau flavanone d'hespéritine (3',5',5-trihydroxy-4'-méthoxyflavanone) auquel est liée de manière covalente une partie glucosidique de rutinose L-rhamnosyl-(α 1→6)-glucose) fixée sur le groupe hydroxyle présent sur le carbone en position 7 de l'hespéritine.

Par hespéridine, on entend ainsi le composé (S)-7[[6-0-(6-deoxy-α-L-mannopyranosyl)-β-D-glucopyranosyl]oxy]-2,3-dihydro-5-hydroxy-2-(3-hydroxy-4-méthoxyphenyl)-4H-1-benzopyran-4-one.

Les dérivés de l'hespéridine peuvent être choisis parmi ses formes aglycones, ses formes chalcones, ses formes glycolysées et ses formes méthylées, ainsi que ses formes sulfatées ou glucuronidées qui sont retrouvées comme produits du métabolisme dans la circulation sanguine.

Les dérivés d'hespéridine peuvent être obtenus par divers procédés connus de l'homme du métier, à l'image par exemple des traitements enzymatiques ou encore être obtenus par synthèse. Le glucose-7-hespéritine peut ainsi être préparé par un traitement par la rhamnosidase ou l'hespéridinase.

A titre de dérivé de l'hespéridine, on peut notamment citer les composés suivants:
- le composé hespérétine, constitué du noyau flavanone non glycosylé de l'hespéridine, qui a la formule suivante: (S)-2,3-dihydro-5,7-dihydroxy-2-(3-hydroxy-4-méthoxyphényl)-4H-1-benzopyran-4-one;3',5,7-trihydroxy 4'-méthoxy flavanone;
- l'α-glucosyl-hespéridine, qui comporte une chaîne de 1 à 20 résidus de glucose liés entre eux par une liaison 1,4, la chaîne de résidus glucose étant liée elle-même par une liaison de type 1,4 en position 4 du résidu glucose de hespéridine; ces dérivés de l'hespéridine et leur procédé de préparation sont décrits notamment dans la demande de brevet EP 0 825 196 et dans le brevet US 6 048 712;
- les composés méthyl-hespéridine, notamment le composé 3¹-méthyl-7-(rhamnosyl-2-méthyl-glucosyl)hespéridine et le composé 3¹-méthylhespéridine, ces composés, ainsi que leur procédé de préparation étant décrits dans le brevet US 858 784;
- les conjugués d'hespérétine et de sulfate ou de glucuronide, qui sont retrouvés, avec l'hespérétine, comme produits de la métabolisation de l'hespéridine dans la circulation sanguine.

Selon un mode de réalisation, on utilise de préférence l'hespéridine et ses dérivés choisis parmi l'hespéritine et le glucuronide d'hespéritine.

D'une manière générale, la quantité efficace d'hespéridine ou de l'un de ses dérivés peut être mise en oeuvre à raison de 0,00001 à 20 % en poids, par exemple de 0,001 à 10 % en poids par rapport au poids total d'une composition en contenant.

Les compositions selon l'invention peuvent se présenter sous toutes les formes galéniques normalement disponibles pour le mode d'administration retenu.

Le support peut être de nature diverse selon le type de composition considérée.

Dans le cas d'une utilisation conforme à l'invention par voie orale, on privilégie l'utilisation d'un support ingérable.

Le support ingérable peut être de nature diverse selon le type de composition considérée.

Conviennent ainsi notamment comme supports alimentaires ou pharmaceutiques des comprimés ou des tablettes, des suppléments oraux sous forme sèche et des suppléments oraux sous forme liquide.

Il peut par exemple s'agir de compléments alimentaires, dont la formulation peut être réalisée par les procédés usuels pour produire notamment des dragées, gélules, gels, émulsions, comprimés, capsules.

Dans le cas de prises par voie orale, les doses journalières peuvent, pour l'hespéridine ou l'un de ses dérivés aller de 0,5 à 2500 mg/j, notamment de 5 à 500 mg/j.

L'hespéridine ou l'un de ses dérivés utilisables selon l'invention peuvent être par ailleurs formulés avec les excipients et composants usuels pour de telles compositions orales ou compléments alimentaires, à savoir notamment composants gras et/ou aqueux, agents humectants, épaississants, conservateurs, agents de texture, de saveur et/ou d'enrobage, antioxydants, conservateurs et colorants usuels dans le domaine de l'alimentaire.

Les agents de formulation et excipients pour composition orale, et notamment pour compléments alimentaires, sont connus dans ce domaine et ne font pas ici l'objet d'une description détaillée.

Quelque soit le mode d'administration considéré, la quantité efficace d'hespéridine ou de l'un de ses dérivés peut également être avantageusement associée à au moins un autre actif.

A titre d'actifs utilisables, on peut citer les vitamines A, B3, B5, B6, B8, C, D, E, ou PP, les curcuminoïdes, les caroténoïdes, les composés polyphénols et minéraux, les sucres, les acides aminés, les acides aminés soufrés, les acides gras polyinsaturés 3 et 6, les probiotiques, la taurine et les phytostérols.

En particulier, on peut utiliser un complexe anti-oxydant comprenant les vitamines C et E, et au moins un caroténoïde, notamment un caroténoïde choisi parmi le β-carotène, le lycopène, l'astaxanthine, la zéaxanthine et la lutéine, des flavonoïdes telles que les catéchines, des proanthocyanidines, des anthocyanines, des ubiquinones, des extraites de café contenant des polyphénols et/ou des diterpènes, des extraits de chicorés, des extraits de ginkgo biloba, des extraits de raisins riches en proanthocyanidines, des extraits de piment, des extraits de soja, d'autres sources de flavonoïdes possédant des propriétés antioxydantes, des acides gras, des prébiotiques, des probiotiques, de la taurine, du resveratrol, des acides aminés du sélénium, des précurseurs de glutathion.

Parmi les flavonoïdes, on choisit de préférence les catéchines et les OPC (oligomères procynidoliques).

Pour ce qui est des actifs lipophiles, on peut utiliser le rétinol (vitamine A) et ses dérivés, le tocophérol (vitamine E) et ses dérivés, les céramides, les huiles essentielles et les insaponifiables (tocotriènol, sésamine, gamma oryzanol, phytostérols, squalènes, cires, terpènes).

Comme actifs susceptibles d'être plus particulièrement associés à l'hespéridine ou à l'un de ses dérivés dans une formule galénique orale, on peut également considérer tous les ingrédients communément utilisés et/ou autorisés, notamment les agents actifs destinés à la prévention et/ou au traitement des affections cutanées.

A titre illustratif, on peut citer les vitamines, les minéraux, les lipides essentiels, les oligoéléments, les polyphénols, les flavonoides, les phytoestrogènes, les antioxydants tels que l'acide lipoïque et le coenzyme Q10, les caroténoïdes, les probiotiques, les prébiotiques, les protéines et les acides aminés, les mono et polysaccharides, les amino-sucres, les phytostérols et alcools triterpéniques d'origine végétale.

Il s'agit, en particulier, des vitamines A, C, D, E, PP et du groupe B. Parmi les caroténoïdes, on choisit de préférence, le béta-carotène, le lycopène, la lutéine, la zéazanthine et l'astaxanthine. Les minéraux et oligo-éléments particulièrement mis en oeuvre sont le zinc, le calcium, le magnésium, le cuivre, le fer, l'iode, le manganèse, le sélénium, le chrome (III). Parmi les composés polyphénols, on retient aussi en particulier les polyphénols de raisin, de thé, d'olive, de cacao, de café, de pomme, de myrtille, de sureau, de fraise, de canneberge, et d'oignon. De préférence parmi les phytoestrogènes, on retient les isoflavones sous la forme libre ou glycosylée, telles que la génistéine, la daidzéine, la glycitéine ou encore les lignanes, en particulier ceux du lin et du schizandra chinensis. Les probiotiques sont choisis de préférence dans le groupe constitué par les lactobacilles et les bifidobactéries. Les acides aminés ou les peptides et les protéines les contenant, tels que la taurine, la thréonine, la cystéine, le tryptophane, la méthionine. Les lipides appartiennent de préférence au groupe des huiles contenant des acides gras mono et polyinsaturés tels que les acides oléique, linoléique, alpha-linolénique, gamma-linolénique, stearidonique, les acides gras oméga-3 de poisson à longue chaîne tels que l'EPA et le DHA, les acides gras conjugués issus de végétaux ou d'animaux tels que les CLA (Conjugated Linoleic Acid).

Ainsi, en particulier lorsque l'hespéridine ou l'un de ses dérivés considérés selon l'invention sont destinés à une administration par voie orale, ils peuvent être associés en outre à au moins un actif nutritionnel choisi parmi le lycopène, la vitamine C, la vitamine E et les composés polyphénols.

En vue d'une utilisation par voie orale, l'hespéridine ou de l'un de ses dérivés peuvent également être associés à d'autres actifs nutritionnels choisis parmi :
- les actifs nutritionnels anti-âge, tels que les antioxydants alimentaires, les nutriments aux propriétés anti-radicalaires et les cofacteurs des enzymes endogènes antioxydants, les vitamines A, C, E, les caroténoïdes, les xantophyles, les isoflavones, certains minéraux tels que le zinc, le cuivre, le magnésium, le sélénium, l'acide lipoïque, le co-enzyme Q10, la superoxyde dismutase (SOD) ou encore la taurine. Parmi les actifs anti-âges, on peut notamment citer les fractions insaponifiables extraits de lipides d'origine végétale, aloe vera, le collagène marin natif ou hydrolysé, les huiles végétales ou marines riches en acides gras oméga-3, en oméga-6 (y compris l'acide gamma-linolénique),
- les actifs nutritionnels photoprotection tels que : les antioxydants et les antiradicalaires, : les vitamines A, C, E, caroténoïdes, xantophyles, certains minéraux tels que le zinc, le cuivre, le magnésium, le sélénium, la co-enzyme Q10, la superoxyde dismultase (SOD), les probiotiques,
- les ingrédients nutritionnels présentant des propriétés d'hydratation ou encore immunomodulatrices tels que l'extrait de polypodium leucotomos, les huiles végétales ou marines riches en acides gras oméga-3, en oméga-6, y compris l'acide gamma-linolénique,
- les actifs nutritionnels actifs sur les signes cliniques de la ménopause (par exemple bouffées de chaleur, ...), tels que les isoflavones, les lignanes, la DHEA, les extraits de yarn, de sauge, de houblon, le calcium, le magnésium, les hydrolysats de protéines, les huiles végétales ou marines riches en acides gras oméga-3,
- les ingrédients nutritionnels mis en oeuvre dans le domaine de la minceur, tels que les extraits de thé vert, maté, marron d'inde, kola, caféine, théobromine, synéphrine, bromelaïne, éphédra, citrus aurantium, calcium, hoodia, garcinia, chitosan, fibres végétales (cactus, pommes, ananas, ...), fenouil, cassis, reine des près, radis noir.

Le procédé de traitement cosmétique de prévention et/ou de traitement des peaux relâchées et/ou des vergetures de l'invention, et en particulier les procédés de traitement cosmétique destinés à atténuer l'aspect de la cellulite et de la peau d'orange, peuvent être mis en oeuvre notamment en appliquant les compositions cosmétiques ou associations telles que définies ci-dessus, selon la technique d'utilisation habituelle de ces compositions.

Selon un mode de réalisation, le procédé de traitement cosmétique selon l'invention peut être précédé d'un régime amincissant.

Le procédé cosmétique selon l'invention peut être mis en oeuvre par administration orale, journalière par exemple, de compositions cosmétiques, ou de l'association selon l'invention qui peut être par exemple formulée sous forme de gels, lotions, émulsions ou supports ingérables.

Le procédé selon l'invention peut comprendre une administration unique.

Selon un autre mode de réalisation, l'administration est répétée par exemple 2 à 3 fois quotidiennement sur une journée ou plus et généralement sur une durée prolongée d'au moins 4 semaines, voire 4 à 15 semaines, avec le cas échéant une ou plusieurs périodes d'interruption.

Dans la description et dans les exemples suivants, sauf indication contraire, les pourcentages sont des pourcentages en poids et les plages de valeurs libellées sous la forme "entre ... et ..." incluent les bornes inférieure et supérieure précisées. Les ingrédients sont mélangés, avant leur mise en forme, dans l'ordre et dans des conditions facilement déterminées par l'homme de l'art.

Les exemples et figures ci-après sont présentés à titre illustratif et non limitatif du domaine de l'invention.
Figure 1 : Elle rend compte de l'expression du collagène IV observée au niveau de la jonction dermo-epidermique chez une population de sujet complémenté oralement en hespéridine vers une population témoin.
Figure 2 : Elle rend compte de l'expression du collagène VII au niveau du derme papillaire chez une population de sujet complémenté oralement en hespéridine vers une population témoin.

### Exemple 1 : Capsule

| | **mg/capsule** |
|---|---|
| Vitamine C | 60 |
| Hespéridine commercialisée par la société SELECTCHEMIE (hespéridine sous forme micronisée pure à 93%) | 15 |
| Glycérine | 150 |
| Stéarate de magnésium | 0,02 |
| Arôme naturel | qsp |

On peut prendre une à trois de ces capsules par jour.

### Exemple 2

On adjoint à la formulation de l'exemple 1 un complexe vitaminique comportant 60 mg de vitamine C, 100 µg de vitamine E et 6 mg de β-caroténe.

### Exemple 3

On adjoint à la formulation de l'exemple 1 un complexe vitaminique comportant 100 mg de vitamine C, 100 µg de vitamine E et 6 mg de lycopène par capsule.

### Exemple 4 : Etude de l'effet de l'hespéridine sur les collagènes IV et VII

L'étude a été réalisée sur des sujets âgés et sains soumis, pendant 24 semaines, à un régime alimentaire comprenant ou non de l'hespéridine.

Le premier groupe de sujets (groupe E) est un groupe témoin soumis à un régime alimentaire standard dépourvu d'hespéridine.

Le deuxième groupe de sujets (groupe F) est un groupe soumis à un régime alimentaire comprenant 0,1 % en poids d'hespéridine par rapport au régime alimentaire standard, ce qui correspond à 50 mg d'hespéridine par kg de poids corporel du sujet et par jour.

Le troisième groupe de sujets (groupe G) est un groupe soumis à un régime alimentaire comprenant 0,5 % en poids d'hespéridine par rapport au régime alimentaire standard, ce qui correspond à 250 mg d'hespéridine par kg de poids corporel du sujet et par jour.

Des échantillons de peau ont été prélevés et analysés afin d'étudier par immunofluorescence l'expression des marqueurs cutanés collagène IV et collagène VII caractéristiques de la jonction dermo-épidermique.

### a) Effet de l'hespéridine sur le collagène IV

### Protocole expérimental

L'immunomarquage du collagène IV est effectué par immunohistochimie avec un anticorps primaire « anticollagen type IV polyclonal » [Rockland (revendu par TEBU-BIO), dilution 1/50] et un anticorps secondaire « anti-lapin FITC » [SANTA CRUZ, dilution 1/100].

Le marquage a été réalisé sur des coupes congelées de 5 µm d'épaisseur issues de prélèvements cutanés.

Les lames ont été récupérées dans du PBS pendant 5 minutes, incubées avec l'anticorps primaire pendant 1 heure à température ambiante dans l'obscurité, rincées dans du PBS à deux reprises pendant 5 minutes, puis incubées avec l'anticorps secondaire pendant 1 heure à température ambiante dans l'obscurité puis à nouveau rincées dans du PBS à deux reprises pendant 5 minutes.

Les lames sont ensuite montées avec du Vectashield (Vector) contenant du DAPI (4,6-diamino-2-phénylindole), puis recouvertes d'une lamelle.

### Résultats

Le marquage collagène IV est localisé dans l'épiderme, exclusivement au niveau de la jonction dermo-épidermique (JDE) et dans le derme, au niveau des structures internes.

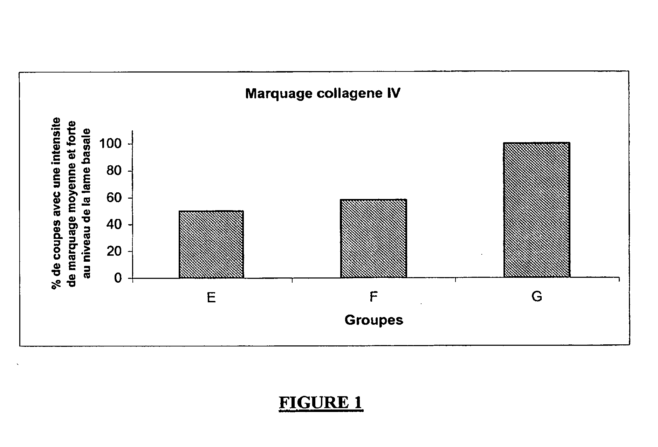

Une expression plus importante du collagène IV est observée dans les deux groupes traités par rapport au témoin (contrôle) au niveau de la lame basale (Fig. 1).

Le tableau ci-dessous résume le nombre de coupes effectuées dans chaque groupe où l'intensité du marquage collagène IV s'est avérée moyenne et forte au niveau de la JDE:

| Groupes | Nombre de coupes (sur 12) avec un marquage moyen et fort de la JDE | % |
|---|---|---|
| E | 6 | 50 |
| F | 7 | 58 |
| G | 12 | 100 |

L'hespéridine permet d'obtenir une restructuration du réseau collagénique au niveau du tissu dermique et une augmentation de l'expression du collagène IV, l'un des marqueurs spécifiques de la jonction dermo-épidermique.

### b) Effet de l'hespéridine sur le collagènes VII

### Protocole expérimental

L'immunomarquage du collagène VII est effectué comme indiqué précédemment mais en utilisant, à titre d'anticorps primaire, « anticollagen type VII polyclonal » [EUROMEDEX (revendu par CHEMICON), dilution 1/500] et, à titre d'anticorps secondaire « anti-1g de souris FITC » [DAKO, dilution 1/100].

### Résultats

Le marquage collagène VII est localisé au niveau de la jonction dermo-épidermique et parfois présent également dans le derme papillaire.

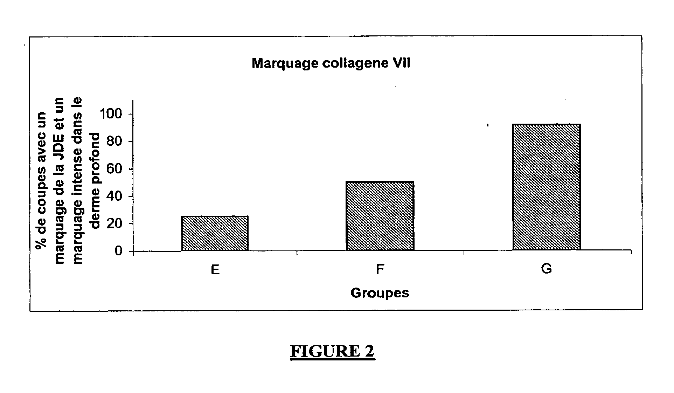

On constate une augmentation de l'intensité de fluorescence plus particulièrement pour le groupe G au niveau de la JDE et du derme papillaire (Fig.2).

Le tableau ci-dessous résume le nombre de coupes effectuées dans chaque groupe avec un marquage collagène VU au niveau de la JDE et au niveau du derme papillaire en fonction des différents traitements et le pourcentage associé :

| Groupes | Nombre de coupes (sur 12) avec um marquage de la JDE et un marquage dans le derme papillaire | % |
|---|---|---|
| E | 3 | 25 |
| F | 6 | 50 |
| G | 11 | 92 |

L'hespéridine permet d'obtenir une restructuration du réseau collagénique au niveau du tissu dermique et une augmentation de l'expression du collagène VII, l'un des marqueurs spécifiques de la jonction dermo-épidermique.

## Revendications

1. Utilisation cosmétique par voie orale d'une quantité efficace d'hespéridine ou de l'un de ses dérivés choisis parmi ses formes aglycones, ses formes chalcones, ses formes glycosylées et ses formes méthylées, ainsi que ses formes sulfatées ou gluçuronidées qui sont retrouvées comme produits du métabolisme dans la circulation sanguine comme agent raffermissant de la peau.

2. Utilisation cosmétique par voie orale d'une quantité efficace d'hespéridine ou de l'un de ses dérivés choisis parmi ses formes aglycones, ses formes chalcones, ses formes glycosylées et ses formes méthylées, ainsi que ses formes sulfatées ou glucuronidées qui sont retrouvées comme produits du métabolisme dans la circulation sanguine comme agent pour prévenir et/ou traiter les peaux relâchées.

3. Utilisation cosmétique par voie orale d'une quantité efficace d'hespéridine ou de l'un de ses dérivés choisis parmi ses formes aglycones, ses formes chalcones, ses formes glycosylées et ses formes méthylées, ainsi que ses formes sulfatées ou glucuronidées qui sont retrouvées comme produits du métabolisme dans la circulation sanguine comme agent pour maintenir et/ou restaurer la tonicité et/ou la fermeté de la peau.

4. Utilisation cosmétique par voie orale d'une quantité efficace d'hespéridine ou de l'un de ses dérivés choisis parmi ses formes aglycones, ses formes chalcones, ses formes glycosylées et ses formes méthylées, ainsi que ses formes sulfatées ou glucuronidées qui sont retrouvées comme produits du métabolisme dans la circulation sanguine comme agent pour atténuer l'aspect de la cellulite et de la peau d'orange.

5. Utilisation cosmétique par voie orale d'une quantité efficace d'hespéridine ou de l'un de ses dérivés choisis parmi ses formes aglycones, ses formes chalcones, ses formes glycosylées et ses formes méthylées, ainsi que ses formes sulfatées ou glucuronidées qui sont retrouvées comme produits du métabolisme dans la circulation sanguine comme agent pour traiter les vergetures ou prévenir leur apparition.

6. Utilisation cosmétique d'une composition comprenant une quantité efficace d'hespéridine ou de l'un de ses dérivés choisis parmi ses formes aglycones, ses formes chalcones, ses formes glycosylées et ses formes méthylées, ainsi que ses formes sulfatées ou glucuronidées qui sont retrouvées comme produits du métabolisme dans la circulation sanguine par voie orale pour prévenir et/ou traiter les peaux relâchées.

7. Utilisation selon la revendication précédente, dans laquelle les dérivés sont obtenus par des traitements enzymatiques ou encore sont obtenus par synthèse.

8. Utilisation selon l'une quelconque des revendications précédentes, **caractérisée en ce qu'**on utilise l'hespéridine et ses dérivés choisis parmi l'hespéritine et le glucuronide d'hespéritine.

9. Utilisation selon l'une quelconque des revendications précédentes, dans laquelle la quantité efficace d'hespéridine ou de l'un de ses dérivés est mise en oeuvre à raison de 0,00001 % à 20 % en poids, par exemple de 0,001 à 10 % en poids par rapport au poids total d'une composition en contenant.

10. Utilisation selon l'une quelconque des revendications précédentes, dans laquelle la quantité efficace d'hespéridine ou de l'un de ses dérivés est associée à au moins un autre actif choisi parmi les vitamines A, B3, B5, B6, B8, C, D, E, ou PP, les curcuminoïdes, les caroténoïdes, les composés polyphénols et minéraux, les sucres, les acides aminés, les acides aminés soufrés, les acides gras polyinsaturés 3 et 6, les probiotiques, la taurine et les phytostérols.

11. Procédé cosmétique de traitement et/ou de prévention des peaux relâchées et/ou des vergetures comprenant l'administration par voie orale à un sujet d'une quantité efficace d'hespéridine ou de l'un de ses dérivés choisis parmi ses formes aglycones, ses formes chalcones, ses formes glycosylées et ses formes méthylées, ainsi que ses formes sulfatées ou glucuronidées qui sont retrouvées comme produits du métabolisme dans la circulation sanguine.

12. Procédé selon la revendication 11, dans lequel ledit sujet est une femme enceinte.

13. Procédé selon la revendication 11, dans lequel ledit sujet est un sujet suivant un régime amincissant.

14. Procédé selon l'une quelconque des revendications 11 à 13, dans lequel la quantité efficace d'hespéridine ou de l'un de ses dérivés est telle que définie en revendication 9.

## Patentansprüche

1. Oral eingenommene, kosmetische Verwendung einer wirksamen Menge von Hesperidin oder eines seiner Derivate als hautfestigender Wirkstoff, gewählt unter seinen aglykonen, chalkonen, glykosylierten und methylierten Formen, sowie seiner sulfatierten oder glucuronidierten Formen, die als Produkte des Metabolismus im Blutkreislauf wiedergefunden werden.

2. Oral eingenommene, kosmetische Verwendung einer wirksamen Menge von Hesperidin oder eines seiner Derivate als Wirkstoff zur Vorbeugung und/oder Behandlung erschlaffter Haut, gewählt unter seinen aglykonen, chalkonen, glykosylierten und methylierten Formen, sowie seiner sulfatierten oder glucuronidierten Formen, die als Produkte des Metabolismus im Blutkreislauf wiedergefunden werden.

3. Oral eingenommene, kosmetische Verwendung einer wirksamen Menge von Hesperidin oder eines seiner Derivate als Wirkstoff zur Erhaltung und/oder Wiederherstellung des Hauttonus und/oder der Hautfestigkeit, gewählt unter seinen aglykonen, chalkonen, glykosylierten und methylierten Formen, sowie seiner sulfatierten oder glucuronidierten Formen, die als Produkte des Metabolismus im Blutkreislauf wiedergefunden werden.

4. Oral eingenommene, kosmetische Verwendung einer wirksamen Menge von Hesperidin oder eines seiner Derivate als Wirkstoff zur Milderung des Erscheinungsbildes der Zellulitis und der Orangenhaut, gewählt unter seinen aglykonen, chalkonen, glykosylierten und methylierten Formen, sowie seiner sulfatierten oder glucuronidierten Formen, die als Produkte des Metabolismus im Blutkreislauf wiedergefunden werden.

5. Oral eingenommene, kosmetische Verwendung einer wirksamen Menge von Hesperidin oder eines seiner Derivate als Wirkstoff zur Behandlung von Dehnungsstreifen oder zur Vorbeugung ihrer Erscheinung, gewählt unter seinen aglykonen, chalkonen, glykosylierten und methylierten Formen, sowie seiner sulfatierten oder glucuronidierten Formen, die als Produkte des Metabolismus im Blutkreislauf wiedergefunden werden.

6. Oral eingenommene, kosmetische Verwendung einer Zusammensetzung umfassend eine wirksame Menge von Hesperidin oder eines seiner Derivate zur Vorbeugung und/oder Behandlung erschlaffter Haut, gewählt unter seinen aglykonen, chalkonen, glykosylierten und methylierten Formen, sowie seiner sulfatierten oder glucuronidierten Formen, die als Produkte des Metabolismus im Blutkreislauf wiedergefunden werden.

7. Verwendung nach dem vorhergehenden Anspruch, bei der die Derivate durch enzymatische Aufbereitung oder durch Synthese erhalten werden.

8. Verwendung nach einem der vorhergehenden Ansprüche, **dadurch gekennzeichnet, dass** das verwendete Hesperidin und seine Derivate unter Hesperitin oder Hesperitin-Glucuronid gewählt wurden.

9. Verwendung nach einem der vorhergehenden Ansprüche, bei der die wirksame Menge von Hesperidin oder eines seiner Derivate zu 0,00001 Gew.-% bis 20 Gew.-%, zum Beispiel 0,001 Gew.-% bis 10 Gew.-% im Verhältnis zum Gesamtgewicht einer die Menge beinhaltendenden Zusammensetzung eingesetzt wird.

10. Verwendung nach einem der vorhergehenden Ansprüche, bei der die wirksame Menge von Hesperidin oder eines seiner Derivate mit mindestens einem anderen Wirkstoff verbunden wird, gewählt aus der Gruppe der Vitamine A, B3, B5, B6, B8, C, D, E oder PP, der Curcuminoide, der Carotinoide, der polyphenolyischen Verbindungen und Mineralien, der Zucker, der Aminosäuren, der schwefelhaltigen Aminosäuren, der mehrfach ungesättigten Fettsäuren 3 und 6, der Probiotika, der Taurine, und der Phytosterole.

11. Kosmetisches Verfahren zur Behandlung und/oder Vorbeugung erschlaffter Haut und/oder Dehnungsstreifen, umfassend die orale Verabreichung an eine Person einer wirksamen Menge von Hesperidin oder eines seiner Derivate, gewählt unter seinen aglykonen, chalkonen, glykosylierten und methylierten Formen, sowie seiner sulfatierten oder glucuronidierten Formen, die als Produkte des Metabolismus im Blutkreislauf wiedergefunden werden.

12. Verfahren nach Anspruch 11, bei dem die Person eine schwangere Frau ist.

13. Verfahren nach Anspruch 11, bei dem die Person eine eine Abmagerungsdiät machende Person ist.

14. Verfahren nach einem der Ansprüche 11 bis 13, bei dem die wirksame Menge von Hesperidin oder eines seiner Derivate der in Anspruch 9 definierten Menge entspricht.

## Claims

1. The cosmetic use via oral route of an effective quantity of hesperidin or one of the derivatives thereof chosen from its aglycone forms, its chalcone forms, its glycosylated forms and its methylated forms, and also its sulfated or glucuronidated forms found as metabolic products in the bloodstream, as skin firming agent.

2. The cosmetic use via oral route of an effective quantity of hesperidin or one of the derivatives thereof chosen from its aglycone forms, its chalcone forms, its glycosylated forms and its methylated forms, and also its sulfated or glucuronidated forms found as metabolic products in the bloodstream, as agent to prevent and/or treat slackened skin.

3. The cosmetic use via oral route of an effective quantity of hesperidin or one of the derivatives thereof chosen from its aglycone forms, its chalcone forms, its glycosylated forms and its methylated forms, and also its sulfated or glucuronidated forms found as metabolic products in the bloodstream, as agent to maintain and/or restore skin tonicity and/or firmness.

4. The cosmetic use via oral route of an effective quantity of hesperidin or one of the derivatives thereof chosen from its aglycone forms, its chalcone forms, its glycosylated forms and its methylated forms, and also its sulfated or glucuronidated forms found as metabolic products in the bloodstream, as agent to attenuate the appearance of cellulite and orange peel skin.

5. The cosmetic use via oral route of an effective quantity of hesperidin or one of the derivatives thereof chosen from its aglycone forms, its chalcone forms, its glycosylated forms and its methylated forms, and also its sulfated or glucuronidated forms found as metabolic products in the bloodstream, as agent to treat stretch marks or to prevent the onset thereof.

6. The cosmetic use of a composition comprising an effective quantity of hesperidin or one of the derivatives thereof chosen from its aglycone forms, its chalcone forms, its glycosylated forms and its methylated forms, and also its sulfated or glucuronidated forms found as metabolic products in the bloodstream, via oral route to prevent and/or treat slackened skin.

7. The use according to the preceding claim wherein the derivatives are obtained by enzymatic treatments or are obtained by synthesis.

8. The use according to any of the preceding claims **characterized in that** hesperidin is used and its derivatives chosen from among hesperitin and hesperitin glucuronide.

9. The use according to any of the preceding claims wherein the effective quantity of hesperidin or one of the derivatives thereof is used in a proportion of 0.00001 % to 20 % by weight, for example from 0.001 to 10 % by weight relative to the total weight of a composition containing the same.

10. The use according to any of the preceding claims wherein the effective quantity of hesperidin or of one of the derivatives thereof is associated with at least one other active ingredient chosen from among vitamins A, B3, B5, B6, B8, C, D, E or PP, curcuminoids, carotenoids, polyphenol and mineral compounds, sugars, amino acids, sulfur-containing amino acids, 3 and 6 polyunsaturated fatty acids, probiotics, taurine and phytosterols.

11. A cosmetic method for treating and/or preventing slackened skin and/or stretch marks comprising the administration to an individual via oral route of an effective quantity of hesperidin or of the derivatives thereof chosen from its aglycone forms, its chalcone forms, its glycosylated forms and its methylated forms, and also its sulfated or glucuronidated forms found as metabolic products in the bloodstream.

12. The method according to claim 11 wherein the said individual is a pregnant woman.

13. The method according to claim 11 wherein the said individual is an individual following a slimming diet.

14. The method according to any of claims 11 to 13 wherein the effective quantity of hesperidin or one of the derivatives thereof is such as defined in claim 9.
